# FASCICULE DE BREVET EUROPEEN

(11) **EP 0 490 757 B1**
(45) Date de publication et mention de la délivrance du brevet: **05.03.1997**
(21) Numéro de dépôt: 91403342.8
(22) Date de dépôt: 10.12.1991
(51) Int. Cl.: G01N 1/12, G21F 7/06, C03B 7/00

(54) **Procédé et dispositif pour le prélèvement automatique d'un échantillon de matériau lors de sa coulée dans un conteneur**
Verfahren und Vorrichtung zur automatischen Probennahme eines Materials während seines Giessens in einen Behälter
Method and device for automatic sampling of material during its pouring into a container

(30) Priorité: 12.12.1990 FR 9015567
(43) Date de publication de la demande: 17.06.1992
(73) Titulaire: COMMISSARIAT A L'ENERGIE ATOMIQUE, 75015 Paris (FR)
(72) Inventeur: Bonniaud, Roger, F-30200 Bagnols sur Ceze (FR); Jouan, Antoine, F-30400 Villeneuve les Avignons (FR); Moncouyoux, Jean-Pierre, F-30200 Bagnols sur Ceze (FR); Saint-Gaudens, Michel, F-30290 Laudun (FR)
(74) Mandataire: Poulin, Gérard

(56) Documents cités:
- FR-A- 363 858
- GB-A- 1 341 555
- US-A- 3 295 171
- US-A- 3 365 953
- US-A- 4 597 562
- PATENT ABSTRACTS OF JAPAN vol. 14, no. 162 (P-1029)29 mars 1990 & JP-A-2 19 740 ( POWER REACTOR & NUCLEAR FUEL DEVICE CORP.) 23.01.90

## Description

L'invention concerne le prélèvement entièrement automatisé d'un échantillon de matériau devant être placé dans un conteneur, lorsqu'aucun dispositif n'a été prévu dans ce but dans le conteneur ou dans les installations de traitement dudit matériau avant sa coulée dans le conteneur. Elle concerne en particulier dans l'industrie nucléaire, des solutions de produits de fission devant être stockées définitivement sous forme de verre. L'invention s'applique de manière générale au prélèvement d'un échantillon de matériau liquide lors de sa coulée dans un conteneur dont l'intérieur se trouve inaccessible pour un opérateur.

### ART ANTERIEUR

L'utilisation de réacteurs nucléaires implique des stockages définitifs des produits de fission usagés. Parmi ceux-ci, des solutions de produits et de déchets radioactifs sont vitrifiées en vue de leur stockage définitif. La dernière phase de cette vitrification consiste généralement à écouler ladite solution dans son conteneur de stockage.

La qualité du verre à stocker doit être garantie par un suivi des nombreux paramètres de fonctionnement de cette opération. On peut citer par exemple : la composition chimique des solutions à vitrifier, les bilans de matières, tous les paramètres technologiques contrôlant le procédé, le temps d'affinage, etc. Les fluctuations possibles de ces paramètres, de part et d'autre des valeurs nominales de référence, sont limitées et définies par des bornes inférieures et supérieures, de telle sorte que la composition du produit reste toujours à l'intérieur d'une fourchette d'acceptabilité. Cette dernière est déterminée par des études de sensibilité en laboratoire et confirmée par des expérimentations technologiques.

Le plus souvent, la qualification de ce principe est souvent obtenue par des essais effectués sur un prototype inactif. Néanmoins, les organismes de sûreté, en l'occurrence l'ANDRA, exigent souvent une vérification active par contrôle direct de la qualité du produit. Ce contrôle peut être fait de manière séquentielle, ou être exigé chaque fois que des variations significatives des paramètres de fonctionnement le justifie.

En conséquence, un contrôle nécessite le prélèvement d'un échantillon de verre. En outre, une telle opération n'est pas facile dans une installation industrielle pour les raisons suivantes :
- L'accès au lieu de prélèvement est en général limité, souvent hors de vision directe, et peu accessible aux télémanipulateurs. En outre, l'utilisation d'engins télécommandés n'est jamais souhaitable en cours de fonctionnement, à cause de la présence de courants induction/haute tension et de fortes intensités de courant électrique.
- La coulée du verre à vitrifier s'effectue en général dans des conditions thermiques défavorables.
- Une étanchéité maximale est requise entre le conteneur et le four d'élaboration du verre, pour limiter l'entraînement par volatilité de composés radioactifs et éviter ainsi la contamination externe du conteneur. Tout système de prélèvement rompant cette étanchéité ne peut pas être utilisé dans ce cas. Dans ces conditions, les dispositifs de prélèvement de verre doivent être intégrés au four d'élaboration, ou intégrés au conteneur.

Différents dispositifs de prélèvement sont connus ou utilisés.

Un premier type de dispositifs consiste à prélever directement dans le conteneur un échantillon solide du verre refroidi. Cet échantillonnage est obtenu par carottage du verre directement dans le conteneur, en utilisant un outil diamanté et un dispositif de coin, permettant ainsi de casser la carotte en fin de coupe. Un tel dispositif a l'avantage de prélever le verre en dehors des opérations de coulée, mais présente les inconvénients suivants. Le dispositif est complexe à mettre en oeuvre et nécessite à lui seul une cellule de travail spécifique. Le carottage se fait en surface et n'est donc représentatif que du verre de fin de coulée. L'opération doit se faire sous l'eau, d'où une lixiviation alterant l'échantillon. Dans ce cas, la surface de l'échantillonnage a été abrasée et est donc peu représentative de l'état de surface réel. De plus, le niveau supérieur du verre dans un conteneur est souvent fracturé. En conséquence, l'obtention d'une carotte monobloc est aléatoire.

Un deuxième type de prélèvement consiste à effectuer ce dernier au niveau du four de fusion. L'opération peut se faire en aspirant par une canne plongeant dans le verre en fusion. L'inconvénient principal du système provient d'une part, de sa complexité et, d'autre part, de sa médiocre représentativité.

Le prélèvement peut également se faire en cours de coulée. Dans ce cas, on intercale entre le four d'élaboration et le creuset, au niveau du tube de liaison, un appareillage représentant un moule en graphite sous le filet de verre.

Par exemple, il a déjà été constitué à cet effet un manège rotatif porteur de trois creusets en graphite disposés à 120°. Un ordonnancement déterminé de la rotation de ce manège permet d'exploiter ce dernier. D'autre part, on connaît également un dispositif de prélèvement par translation où un vérin amène un moule en graphite sous la coulée. Ces dispositifs permettent de prélèver plusieurs échantillons à chaque coulée et à plusieurs niveaux de remplissage du conteneur. Par contre, il représente un investissement considérable ne se justifiant que si le nombre d'échantillons est élevé. Ils doivent être prévus dès la conception du four. De plus, le refroidissement de l'échantillon est très rapide et provoque en général la fracturation de ce dernier et évite l'évolution de la cristallisation éventuelle. Dans ces conditions, l'échantillon n'est pas toujours parfaitement représentatif de l'état du verre du conteneur. Un dispositif de refroidissement contrôlé peut palier cet inconvénient au prix d'une complexité accrue, par exemple un four gradient simulant les conditions de refroidissement du verre réel.

Tous ces dispositifs peuvent apporter une solution satisfaisante dans la mesure où ils ont été prévus à la conception de l'installation et en particulier du four, si le coût de cette implantation est rentabilisé par un programme suffisant de prélèvement.

Par exemple, le document japonais JP-A-219 740 décrit un dispositif de prélèvement de verre fondu. Il décrit de manière très succincte un bras muni d'un godet de prélèvement. Cet ensemble est placé en dessous du four où le verre est fondu, sur la trajectoire de ce dernier et au-dessus d'un réservoir de réception. L'ensemble est actionné manuellement ou à l'aide d'un vérin au moyen de la pièce (7).

D'autre part, le document français FR-A-3 363 858 décrit un appareil pour trancher le verre fondu ou le plastique. Il comprend un godet de réception monté au bout d'un levier et pivotant autour d'un axe, l'autre extrémité du levier étant équipée d'un contrepoids. Le godet est placé en-dessous d'un orifice d'un réservoir où se trouve le verre à prélever. L'ensemble est monté pivotant autour d'un axe vertical.

### RESUME DE L'INVENTION

L'invention vise à remédier à l'inconvénient ci-dessus mentionné en proposant un procédé et des dispositifs de prélèvement d'un échantillon pouvant s'adapter au matériel de vitrification et de stockage de verre radioactif existant déjà. L'objectif du prélèvement est souvent dans ce cas la vérification directe et le suivi des paramètres de fonctionnement. Lorsque ce prélèvement s'effectue au niveau du conteneur, il évite les risques associés à la complexité d'un appareillage intégré au four de fusion.

A cet effet, le premier objet principal de l'invention est un procédé de prélèvement automatique d'un échantillon de matériau se trouvant au moins momentanément en phase liquide pour être coulé dans un conteneur et devant être enfermé dans ce conteneur. Selon l'invention, le procédé consiste à :
- recueillir l'échantillon lors de la coulée du matériau dans le conteneur, à l'aide d'un dispositif selon l'une des revendications 3 à 11 fixé au rebord supérieur du conteneur et présentant un godet sous le filet de matériau liquide ; et
- récupérer l'échantillon recueilli dans le godet.

L'échantillon peut ainsi être pris quand la vitesse de coulée est stabilisée et linéaire.

La principale application d'un tel procédé se fait pour le verre radioactif avant son confinement définitif dans un conteneur. Dans ce cas, le dispositif utilisé est laissé définitivement dans le conteneur et y est enfermé avec le produit vitrifié.

Un deuxième objet principal de l'invention est un dispositif pour recueillir automatiquement un échantillon de matériau se trouvant au moins momentanément en phase liquide et étant coulé dans un conteneur.

Selon l'invention, le dispositif comprend :
- un godet pour recueillir le matériau ;
- un bras à une extrémité duquel est fixé le godet ;
- une tige support sur laquelle le bras est monté ; et
- des moyens de fixation au rebord supérieur du conteneur de la tige support pour que le godet puisse se trouver sous le filet de coulée et recueillir l'échantillon.

Dans la réalisation principale de l'invention, le bras est monté oscillant autour d'un axe horizontal et d'un axe vertical et le dispositif comprend des moyens pour commander et effectuer deux rotations du bras. Dans ce cas, la première rotation du bras autour de l'axe vertical se fait d'une position initiale écartée du filet de matériau liquide vers une position de prélèvement du godet sous le filet de matériau liquide ; la deuxième rotation du bras autour de l'axe vertical s'effectue de la position de prélèvement vers une position de repli écartée du filet de matériau liquide.

Dans la réalisation préférentielle des moyens de commande, ceux-ci sont réalisés en partie par une pastille fusible sur laquelle repose une tige lestée bloquant en position initiale le bras et le libérant en rotation par la chute de la tige lestée lors de la fusion de la pastille à une température déterminée.

La première mise en rotation horizontale du bras peut être obtenue de deux manières différentes.

La première consiste à utiliser un ressort hélicoïdal précontraint et monté autour de la tige support, une première extrémité étant fixée à la tige, la deuxième extrémité étant fixée au bras, pour provoquer ainsi la rotation du bras autour de l'axe vertical. La deuxième consiste à utiliser un contrepoids de rotation suspendu et relié horizontalement au bras par un câble passant par des poulies.

La mise en rotation du bras autour de l'axe horizontal est de préférence commandée par un contrepoids placé à la deuxième extrémité du bras pour maintenir dans une position haute de remplissage le godet et pour faire osciller le bras lorsque le godet est rempli du matériau liquide et dégager le bras d'une butée fixe en rotation, libérant ainsi en rotation le bras jusqu'à la position de repli.

La forme préférentielle du godet est telle que celui-ci présente deux compartiments superposés.

Un autre objet principal de l'invention est un dispositif pour récupérer le godet contenant le matériau recueilli après refroidissement du dispositif qui vient d'être résumé.

Selon l'invention, ce dispositif comprend :
- des moyens de positionnement sur le rebord supérieur du conteneur et auquel est montée une pince comportant plusieurs griffes maintenues en position de fermeture par des ressorts de rappel pour pouvoir prendre deux positions verticales différentes qui sont les suivantes :
   . une position haute de repos, les griffes étant fermées ; et
   . une position basse de préhension, les griffes ayant été ouvertes lors de la descente de la pince pour saisir le godet par un rebord supérieur de ce dernier.

Ces deux dispositifs se complètent avantageusement d'un troisième dispositif pour ramener le bras du dispositif pour recueillir l'échantillon dans sa position de prélèvement avant la récupération du godet comprenant :
- des moyens de fixation sur le rebord supérieur du conteneur avec un indexage angulaire, sur lesquels :
- une première tige verticale d'entraînement qui est montée pivotante par rapport à un axe vertical pour faire effectuer au bras oscillant du dispositif pour recueillir l'échantillon une rotation de sa position de repli vers sa position de récupération, par entraînement de l'extrémité inférieure de la première tige verticale d'entraînement sur l'extrémité supérieure d'une deuxième tige verticale d'entraînement du bras oscillant du dispositif pour recueillir l'échantillon.

L'ensemble de ces trois dispositifs est particulièrement bien adapté pour effectuer le prélèvement d'un échantillon de verre radioactif, lors de sa coulée dans son conteneur de stockage.

### LISTE DES FIGURES

La description détaillée suivante de l'invention est annexée de plusieurs figures qui représentent respectivement :
- figures 1A, 1B, 1C, en coupe, le dispositif pour recueillir l'échantillon, avant, pendant et après le remplissage du godet de ce dispositif selon l'invention ;
- figure 2, en coupe partielle, une première réalisation du dispositif pour recueillir l'échantillon selon l'invention ;
- figure 3, en coupe partielle, une deuxième réalisation du dispositif pour recueillir l'échantillon selon l'invention ;
- figures 4A et 4B, en coupe, le dispositif pour ramener le bras du dispositif de la figure 3 dans sa position de prélèvement ; et
- figures 5 et 6, en coupe, le dispositif pour récupérer l'échantillon selon l'invention dans le godet du dispositif de la figure 3, avant et après cette récupération.

### DESCRIPTION DETAILLEE DES REALISATIONS DE L'INVENTION

### Procédé selon l'invention

Le concept de l'invention est basé sur le fait qu'il doit être possible d'effectuer des prélèvements, en particulier de verre radioactif, sur des installations existant déjà. Les dispositifs selon l'invention et décrits ultérieurement ne sont donc pas fabriqués en même temps que le conteneur dans lequel ou sur lequel ils sont installés, encore moins en même temps que le four de fusion du verre.

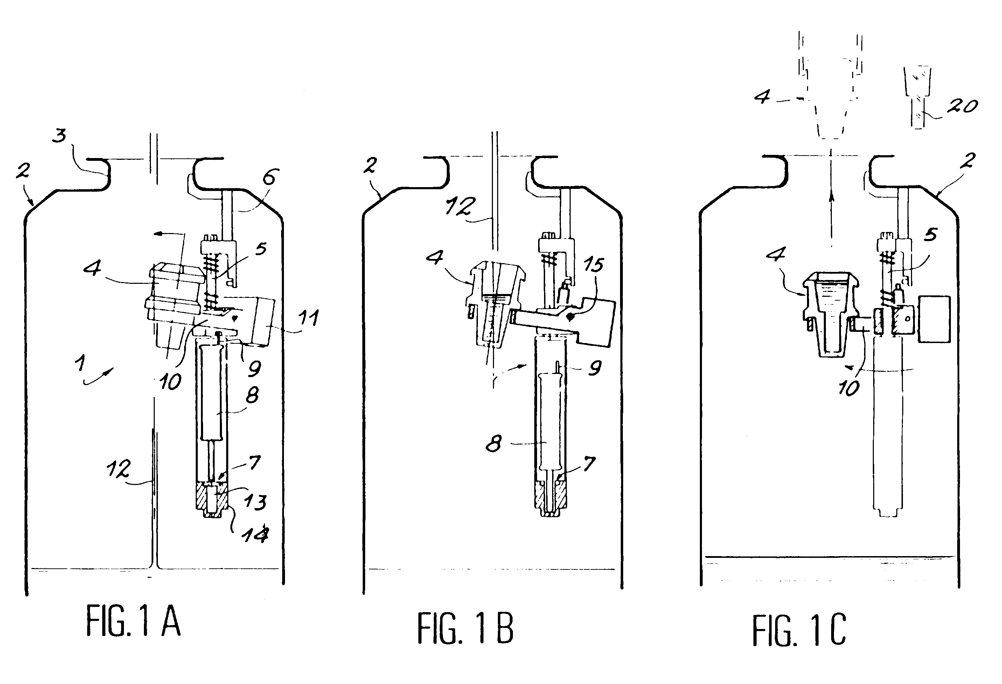

La première phase principale du procédé consiste à prélever l'échantillon du verre au cours de sa coulée dans son conteneur. Ceci implique bien évidemment que le procédé selon l'invention s'applique à un matériau se trouvant au moment du remplissage du conteneur, dans lequel il doit être mis, sous forme liquide. On pense en particulier aux verres radioactifs qui sortent en phase liquide du four de fusion et qui sont coulés directement dans leur conteneur de stockage. Dans ce but, et en référence aux figures 1A, 1B et 1C, on utilise un dispositif fixé au rebord supérieur 3 du conteneur 2 dans lequel le matériau est coulé. Ce dispositif comprend en particulier un godet 4 qui sera amené sous le filet de coulée 12 du matériau.

La deuxième phase principale consiste à récupérer l'échantillon recueilli dans le godet 4, de préférence lorsque le matériau est solidifié. Deux autres dispositifs peuvent alors être utilisés pour effectuer cette opération.

Dans le cas d'un produit radioactif, le dispositif installé au préalable dans le conteneur pour prélever l'échantillon y est laissé définitivement et y est enfermé avec le produit vitrifié. On évite ainsi les risques associés à la contamination du matériel ayant été en contact avec le produit radioactif.

Dans le cas d'un verre radioactif, la fusion de ce dernier peut être effectuée à une température avoisinant 900°C. Il est alors nécessaire d'attendre plusieurs heures pour que le verre se refroidisse et se solidifie.

### Dispositif pour recueillir l'échantillon

En référence à la figure 1A, ce dispositif 1 se compose principalement de :
- moyens de fixation 6 du dispositif 1 au rebord supérieur 3 du conteneur 2 ;
- une tige verticale 5 fixée à ces moyens de fixation 6 ;
- un bras oscillant 10 porteur à une première extrémité du godet 4 et à une autre extrémité d'un contrepoids 11 ;
- un fourreau de protection à l'intérieur duquel se trouvent une tige lestée 8 en appui sur pastille fusible 7 ; et
- un pion 9 pour bloquer en rotation l'arbre oscillant 10.

Cet agencement prévoit que le godet 4 se trouve avant le prélèvement dans une position écartée du filet de coulée 12. Dans ce but, le bras oscillant 10 est monté mobile en rotation horizontale autour de la tige verticale 5. Il peut ainsi prendre une position angulairement décalée par rapport au plan des figures. Pour visualiser cette position, le contrepoids 11 a été représenté en perspective et le godet 4 ne se trouve pas sous le filet de coulée 12.

Lors du remplissage du conteneur 2 le fluide tombe à l'intérieur de celui-ci et le niveau du liquide monte au fur et à mesure. Compte tenu de la température encore élevée du liquide, la température à l'intérieur du conteneur 2 monte et en particulier à un endroit fixe où se trouve la pastille fusible 7. En effet, celle-ci est placée à un endroit déterminé pour qu'elle fonde au moment où on désire effectuer le remplissage du godet 4. Sa hauteur dans le conteneur 2 est donc déterminée. Cette pastille fusible 7 constitue un moyen avantageux de commander la rotation du bras oscillant 10 pour mettre le godet 4 sous le filet de coulée 12.

En effet, en se référant à la figure 1B, la pastille 7 a été représentée fondue, de sorte que la tige lestée 8 est tombée, grâce à un trou 13 pratiqué dans une cellule 14 à l'intérieur de laquelle est placée la pastille fusible 7. En conséquence, le pion 9 qui est solidaire de cette tige lestée 8 est également descendu.

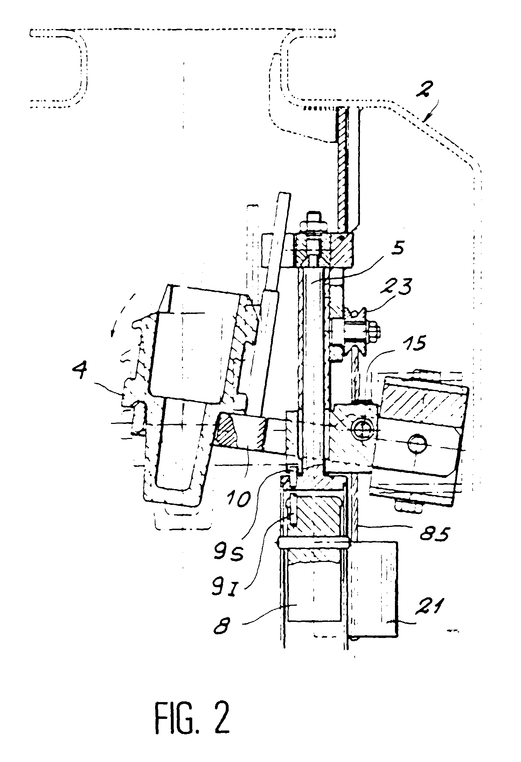

Comme on le voit plus en détail sur la figure 2, ce pion 9 a une partie supérieure 9S insérée dans la tige 5 fixe et le bras oscillant 10 mobile. D'autre part, il a sa partie inférieure 91 fixée dans la tige lestée 8. De la sorte, lorsque cette dernière descend par pénétration dans le trou 13 de la cellule 14, la partie supérieure 9S se trouve sortie en dehors du bras oscillant 10 et de la tige 5. Ces deux derniers organes se trouvent alors libérés en rotation, et le bras 10 peut alors tourner autour de l'axe vertical de la tige 5.

Cette rotation permet au godet 4 d'être positionné en dessous du filet de coulée 12 de manière à recueillir une quantité déterminée de liquide.

Le dispositif selon l'invention possède également dans sa réalisation préférentielle des moyens pour commander une deuxième rotation horizontale, de cette position de prélèvement vers une position de repli, une fois que le godet 4 est rempli. Dans cette position de repli, le godet 4 est écarté du filet de coulée 12. Une manière avantageuse d'obtenir cette deuxième rotation consiste à équiper le bras oscillant 10 d'un contrepoids 11 placé de manière opposée au godet 4. Ce contrepoids doit équilibrer sensiblement la masse de liquide contenue dans le godet 4. De cette manière, lorsque ce dernier est rempli, le bras oscillant 10, sous l'action du poids du liquide prélevé, bascule légèrement autour d'un axe perpendiculaire à l'axe vertical. Dans ce but, l'arbre oscillant 10 est également monté tournant autour d'un arbre horizontal 15 monté dans une noix 16 solidaire de la tige verticale 5. Le contrepoids 11 doit être calculé pour que ce basculement ait bien lieu une fois que le godet 4 est plein de liquide à prélever.

Ce basculement du bras oscillant 10 permet de dégager une butée 17 solidaire du bras oscillant 10 d'une fente 18 pratiquée dans une pièce de butée 19 montée fixe par rapport à la tige verticale 5, c'est-à-dire par rapport au moyen de fixation 6. Cette libération permet à l'arbre oscillant 10 d'effectuer la deuxième rotation horizontale pour dégager le godet 4 du filet de coulée 12. Cette opération est schématisée à la figure 1C sur laquelle le bras oscillant 10 est représenté horizontal, le godet 4 étant rempli.

Sur cette même figure 1C, est suggérée, en traits mixtes, la deuxième phase principale du procédé selon l'invention, c'est-à-dire le retrait du godet 4 en dehors du conteneur 2 et l'extraction de l'échantillon 20 de verre solidifié dans le godet 4.

Les positions initiales de prélèvement et de repli du godet 4 sont donc obtenues par des rotations horizontales du bras oscillant 10 autour de l'axe vertical de la tige 5. La présente description propose deux réalisations des moyens de mise en rotation du bras oscillant 10 autour de la tige verticale 5.

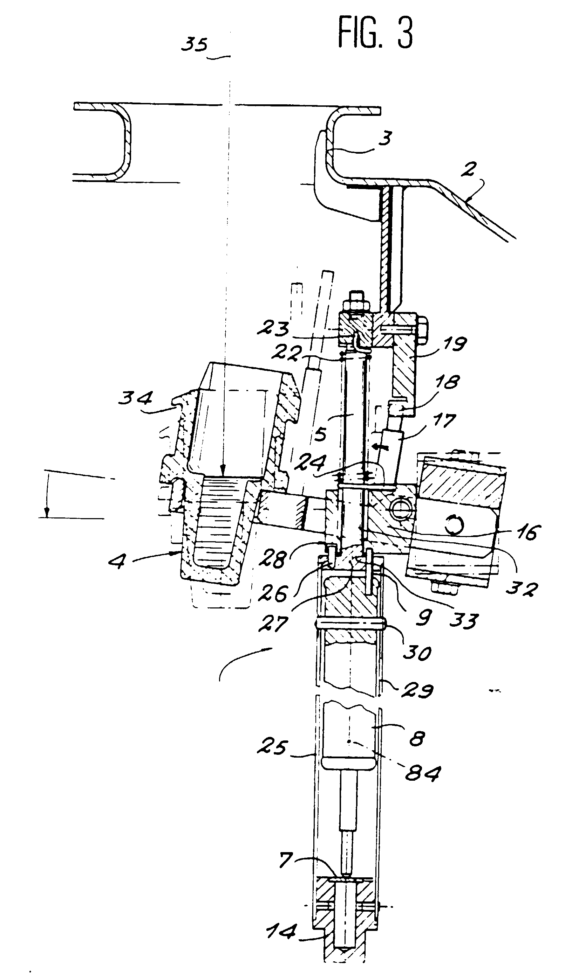

En référence à la figure 2, la première réalisation consiste à utiliser un contrepoids de rotation 21 suspendu à un câble 85 passant autour d'une poulie 23 fixe par rapport à la tige verticale 5. Ce câble 85 a son extrémité non représentée fixée au bras oscillant 10 ou à une pièce solidaire de ce dernier, par exemple la noix 16. Le câble 85 est orienté par rapport au bras oscillant 10 de manière à ce que le contrepoids de rotation 21 exerce une réaction sur ce bras oscillant 10, de manière désaxée par rapport à l'axe de la tige verticale 5. Dans ce cas, lorsque le bras oscillant est libéré du pion 9 le bloquant en rotation dans la position initiale, celui-ci subit la pression du câble 85 et effectue une rotation pour venir positionner le godet en dessous du filet de coulée 12. Cette position est déterminée par la position de la butée 17 dans la fente 18 de la figure 3.

Le contrepoids de rotation 21 agit de même dans le même sens sur le bras oscillant 10 pour faire passer le godet 4 de la position de prélèvement à la position de repli, lorsque le bras oscillant 10 s'est dégagé par basculement de la pièce de butée 19.

Comme le représente la figure 3, et comme suggéré sur les figures 1A, 1B et 1C, la deuxième réalisation des moyens de mise en rotation consiste à utiliser un ressort hélicoïdal 22 placé autour de la tige verticale 5. Son extrémité supérieure 23 est fixée à la tige 5 ou au moyen de fixation 6. L'extrémité inférieure 24 du ressort 22 est fixée au bras oscillant 10. Le ressort 22 est précontraint de sorte que, lorsque ce dernier est libre en rotation, le ressort hélicoïdal 22 a tendance à faire tourner le bras oscillant 10 pour le faire passer de sa position initiale à la position de prélèvement, puis quand le bras oscillant 10 a basculé, à le faire passer de sa position de prélèvement à la position de repli.

Le pion 9 est solidaire de la tige lestée 8 et traverse la base de la tige verticale 6 pour pénétrer dans la noix 16 portant le bras oscillant 10. La chute de la tige lestée 8 oblige donc le pion 9 a se désengager de cette noix 16. Cette dernière est libérée en rotation et peut donc tourner avec le bras oscillant 10. D'autre part, un pion de butée 26 est solidaire de la partie supérieure 27 du fourreau 25. Il pénètre dans une lumière circulaire 28 pratiquée dans la noix 16. La lumière circulaire correspond à la course angulaire entre la position initiale de repos et la position de prélèvement. Ceci permet donc d'arrêter la première rotation du bras oscillant 10 pour placer le godet 4 dans sa position de prélèvement.

Le fourreau 25 est solidaire de la tige verticale 5 et maintient la cellule 14 dans laquelle se trouve la pastille fusible 7. Il leur sert également de boîtier et protège celles-ci des dépôts éventuels de produit dûs à la volabilité du liquide versé dans le conteneur. Ce fourreau 25 comporte dans sa partie supérieure deux lumières 29 destinées à guider la tige lestée 8, par l'intermédiaire d'une goupille transversale 30 débouchant dans ces deux lumières 29. Le rôle de cette dernière est de maintenir la tige lestée 8 dans sa position angulaire lors du montage de la partie lestée 8 dans le fourreau 25.

Des détails concernant l'utilisation de ce dispositif pour recueillir un échantillon de verre radioactif lors de la coulée de ce verre dans son conteneur de stockage définitif, sont maintenant donnés à titre explicatif, mais non limitatif.

On signale tout d'abord que le dispositif peut être utilisé à un moment où la vitesse de coulée peut être de l'ordre de 400 kg de verre par heure.

Ce dispositif reste entièrement automatique et ne nécessite aucune intervention humaine et aucune modification des cotes géométriques du conteneur. En effet, la première rotation de l'ensemble du bras oscillant 10 et du godet 4 est commandée par l'évolution de la température à l'intérieur du conteneur 2. A cet effet, la pastille fusible 7 est placée à une hauteur déterminée de manière très précise.

Avec un débit de coulée de 400 kg par heure, le remplissage d'un godet de 450 grammes est obtenu en 4 secondes. Le dipositif utilisé répond donc très rapidement et de façon parfaitement reproductible.

Le ressort hélicoïdal 22 utilisé, est de préférence du type à boudins fonctionnant en torsion. Il est bandé au moment de la mise en place du dispositif. Sa force de rappel doit être suffisante pour assurer les deux rotations horizontales du du godet 4 jusqu'à la position de repli. On note que le ressort 22 agit sous des conditions thermiques telles qu'il doit supporter une température de 500°C. En conséquence, le matériau utilisé pour élaborer un tel ressort de rappel peut être l'Inconel 601. Le ressort 22 peut avantageusement comporter 38 spires pour un diamètre nominal de 20 mm.

Dans le cadre de la coulée de verre radioactif dans un conteneur 2 de diamètre extérieur, légèrement inférieur à 500 mm et de hauteur supérieure à 1 m, ce dernier est préchauffé à sa base à une température de 500°C. En conséquence, le matériau constituant la pastille fusible 7 doit répondre à certains critères :
- la température de fusion doit être supérieure à cette température de préchauffage du conteneur 2 ;
- la température de fusion doit être celle d'un élément pur ou d'un élément eutectique ou d'un alliage dont l'intervalle de fusion est relativement très restreint ; et
- la conception et le matériau constituant la cellule porte-fusible 14 doivent être tels que l'échange thermique vers la pastille fusible 7 est favorisé pour ne pas entraîner de retard à la fusion ; en conséquence cette cellule 14 est faite d'un matériau le plus léger possible pour ne présenter qu'une charge calorifique faible et les contacts de celle-ci avec la pastille fusible sont suffisants. Compte tenu de ces exigences, la pastille fusible 7 a été placée à 400 mm du fond, et sa température de fusion est de 600°C. Le matériau utilisé peut être un alliage d'aluminium AG₃, un alliage d'aluminium-lithium, des brasures à l'argent ou des alliages cuivre-zinc ou cuivre-étain. On note que la brasure à l'argent du type "Castellin 1802", fabriquée par la Société Française Térotechnologique, répond parfaitement à ces conditions de fonctionnement.

Le bras oscillant 10 peut être en acier inoxydable.

La noix 16 de ce bras oscillant 10 peut porter un palier de graphite, repéré 32 sur la figure 3, et s'appuyer sur une bague de graphite 33 pour faciliter la rotation autour de la tige verticale 5.

Lorsque du verre radioactif, fondu dans les normes de retraitement des déchets nucléaires, est versé dans un conteneur, la température de surface du verre oscille entre 800 et 900°C. Le dispositif pour recueillir l'échantillon doit néanmoins être positionné assez près de la buse de coulée qui déverse le verre liquide, pour éviter que le filet de coulée ne puisse s'écarter du conteneur par sa dérive angulaire et pour faciliter ensuite la récupération du godet 4.

La température ambiante au niveau du dispositif ne doit pas être trop élevée pour limiter les forces de frottement, mais rester à une température suffisante après le prélèvement de l'échantillon, pour que le verre subisse une cinétique de refroidissement suffisamment lente, en particulier aux alentours et en dessous du point de transformation du verre. Une vitesse de refroidissement de l'ordre de 75°C par heure, à partir de 550°C, est acceptable pour éviter la fracturation de l'échantillon prélevé. Compte tenu des rayonnements thermiques dans le volume à l'intérieur du conteneur 2, dans le dispositif selon l'invention, dans les parois du conteneur 2 et dans le godet 4, la position de ce dernier peut être localisée à 925 mm du fond du conteneur 2.

Pour faciliter la réception et le retrait de l'échantillon de matériau prélevé, le godet 4 est prévu avec deux compartiments différents superposés, le compartiment supérieur étant de diamètre plus large que celui du compartiment inférieur.

Pour faciliter le prélèvement futur du godet 4 par rapport au dispositif, un rebord 34 est prévu à l'extérieur de la partie supérieure du godet 4.

Le débattement des déplacements du godet 4 s'effectue sur environ 90°.

La tige verticale 35, solidaire du bras oscillant 10, sert ultérieurement, juste avant l'ablation du godet 4.

Les compartiments du godet 4 ont une légère dépouille verticale pour faciliter l'ablation de l'échantillon solidifié.

### Dispositif pour ramener le bras oscillant dans sa position initiale

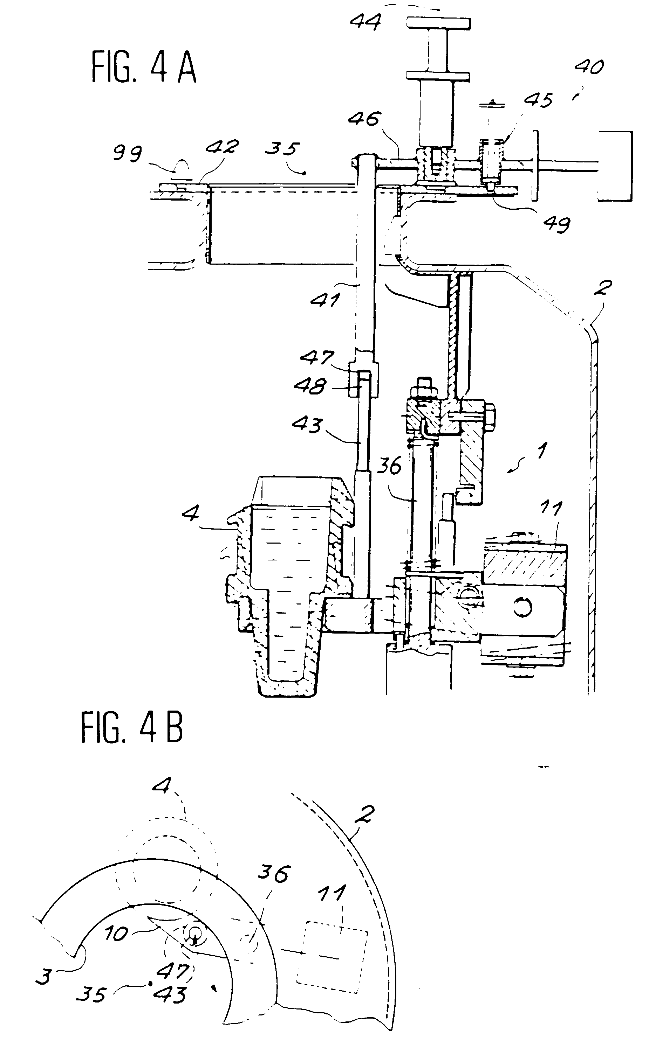

Sur la figure 4B, on distingue, en vue de dessus l'ouverture supérieure du conteneur 2 à l'intérieur duquel se trouve le dispositif qui vient d'être décrit. On distingue en particulier le godet 4, l'axe de pivotement 36 du bras oscillant 10. Le godet 4 est en position de repli, c'est-à-dire qu'il se trouve en dehors de l'ouverture du conteneur 4. En référence aux figures 4A et 4B, on a également prévu un dispositif 40 destiné à ramener le bras oscillant 10 dans sa position de prélèvement, après le cycle de remplissage du godet 4, pour récupérer l'échantillon. Il se compose principalement d'un bras tournant 46 pivotant autour d'un axe vertical 44 et à une extrémité duquel se trouve une première tige verticale d'entraînement 41. Ce dispositif 40 est fixé sur le rebord supérieur du conteneur 2 avec des moyens de fixation qui peuvent être réalisés sous la forme d'un corps 42 s'emboîtant dans l'ouverture du conteneur 2. Un indexage angulaire est prévu au moyen de pions d'indexage 99. Une fois que ce dispositif 40 est fixé sur le conteneur 2, l'extrémité inférieure 47 de la première tige d'entraînement 41 se trouve contre l'extrémité supérieure 48 d'une deuxième tige d'entraînement 43, qui est solidaire de l'arbre oscillant 10.

Une rotation de ce dispositif autour de l'axe vertical 44 entraîne la rotation du bras oscillant 10 autour de l'axe vertical de la tige verticale 5. Le dispositif comportant le godet 4, peut donc être déplacé pour que ce dernier retrouve sa position de prélèvement. Dans l'application au verre radioactif décrite précédemment, le débattement d'un tel dispositif dépasse 60°.

Sur la figure 4B, on distingue également la deuxième tige d'entraînement 43 placée sur l'arbre oscillant 10. La course angulaire de ce bras tournant 46 est limitée par une butée qui est symbolisée sur la figure 4A par une butée à ressort 45, dont l'extrémité pénètre dans un trou 49 du rebord du cylindre de fixation 42.

Une fois que le godet 4 est remis dans sa position de prélèvement, la récupération de l'échantillon par retrait du godet 4 peut avoir lieu.

### Dispositif pour récupérer le godet

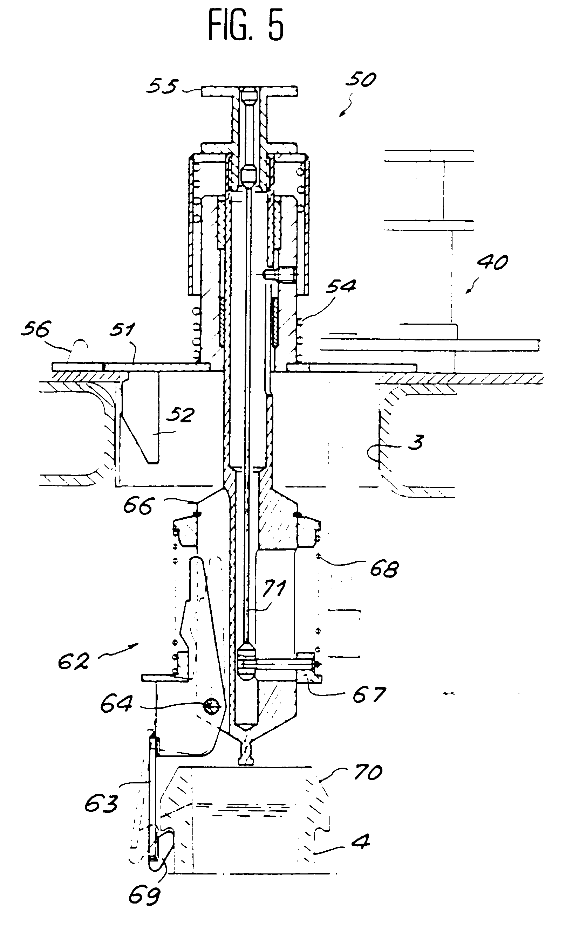

En référence à la figure 5, le dispositif destiné à récupérer le godet 4 comprend principalement des moyens de fixation sur le rebord 3 du conteneur et une pince 62.

Les moyens de fixation comprennent une plaque horizontale 51 sur laquelle sont fixés verticalement des plats de centrage 52. Des pions de centrage 56 sont également fixés sur la plaque horizontale 51.

La pince 62 est constituée de plusieurs griffes 63 montées pivotantes autour d'axes 64 horizontaux sur un corps de pince 66. La préhension du godet 4 se fait par l'intermédiaire des extrémités inférieures 69 des griffes 63. Les griffes 63 sont maintenues en position fermée par des ressorts de rappel non représentés.

La pince 62 est maintenue en position haute par un ressort de rappel 54 prenant appui sur la plaque de fixation 51 et supportant une poignée 55 solidaire de la pince 62. Une couronne 67 entoure les griffes 63 pour les maintenir fermées. Pour récupérer le godet 4, une pression verticale doit être exercée sur la poignée 55 pour faire descendre celle-ci ainsi que la pince 62. Une couronne usinée 70 sur le rebord supérieur du godet 4 écarte alors les griffes 63. La couronne 67 remonte par glissement le long des griffes 63. A la fin de la descente, les griffes se referment sous l'action de leur ressort de rappel respectif.

Le corps 66 de la pince 62 est creux et comporte une tige 71 solidaire de la couronne 67. Elle assure un rôle de témoin en indiquant, par son déplacement, que les griffes 63 se sont refermées correctement à la descente de la pince 62. Le déplacement de la couronne 67 est obtenue grâce à un deuxième ressort de rappel 68 entourant le corps 66.

La remontée de la pince 62 et du godet s'effectue par relâchement de la pression sur la poignée 55.

La manipulation de la pince 62 se fait, dans le cas de verre radioactif, par l'intermédiaire d'un télémanipulateur.

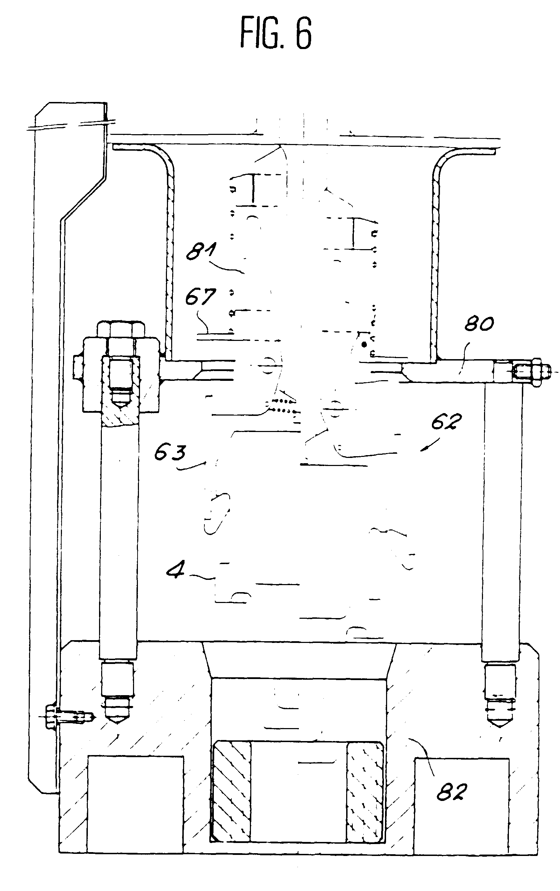

Toujours dans le cas de l'application de l'invention au verre radioactif, il est également nécessaire de disposer d'un appareil du genre télémanipulateur pour libérer le godet 4 de la pince 62. En se référant à la figure 6, l'ensemble de la pince 62 et du godet 4 est placé sur un support 80. Une pression sur la poignée 55 permet d'écarter les griffes 63 par une légère remontée de la couronne 67 contre les bossages 81 des griffes 63. On note que la force nécessaire pour provoquer cette ouverture peut être celle du propre poids de l'ensemble.

Une fois libéré, le godet 4 tombe dans une pièce d'appui 82. La pince 62 est alors enlevée à l'aide du télémanipulateur.

La libération de l'échantillon qui se trouve dans le godet 4, peut être obtenue par retournement de l'ensemble. L'échantillon est alors mis dans un conteneur approprié en vue de son expédition au service de caractérisation devant effectuer des analyses.

Le dispositif de prélèvement de l'échantillon à l'aide du godet 4 doit préférentiellement n'être utilisé qu'une fois, lorsqu'il s'agit de verre radioactif.

Le procédé et les dispositifs selon l'invention permettent le prélèvement de verre radioactif directement dans le conteneur de stockage, sans aucune intervention humaine, lorsque le système intégré au four n'a pas été prévu. Il permet donc la récupération d'éprouvettes de verre sous une forme appropriée pour les analyses. Sa commande est automatiquement réglée par l'évolution thermique de la coulée.

On note que la position de repli du bras oscillant 10 permet d'obtenir une cinématique de refroidissement acceptable pour l'échantillon.

## Revendications

1. Procédé de prélèvement automatique d'un échantillon de matériau se trouvant au moins momentanément en phase liquide pour être coulé dans un conteneur (2) et devant être enfermé dans ce conteneur (2), caractérisé en ce qu'il consiste à effectuer automatiquement les opérations suivantes :
- recueillir l'échantillon lors de la coulée du matériau dans le conteneur (2) à l'aide d'un dispositif selon l'une des revendications 3 à 11 fixé au rebord supérieur (3) du conteneur (2) en présentant un godet (4) sous le filet de matériau liquide (12) ; et
- récupérer l'échantillon recueilli dans le godet (4).

2. Procédé selon la revendication 1, destiné à prélever un échantillon de verre radioactif avant son confinement définitif dans le conteneur (2), le dispositif de prélèvement par un godet (4) étant définitivement abandonné dans le conteneur (2) pour qu'il y soit enfermé avec le produit vitrifié.

3. Dispositif pour recueillir un échantillon de matériau se trouvant au moins momentanément en phase liquide et étant coulé dans un conteneur (2) comprenant un godet (4) pour recueillir le matériau, caractérisé en ce qu'il comprend pour fonctionner automatiquement :
- une tige support (5) ;
- un bras oscillant (10) à une première extrémité duquel est fixé le godet (4), ledit bras étant monté oscillant sur la tige support (5) autour d'un axe horizontal (15) et d'un axe vertical de la tige verticale (5) ;
- des moyens de fixation de la tige support (5) au rebord supérieur (3) du conteneur (2), pour que le godet (4) puisse se trouver sous le filet de coulée (12) et recueillir l'échantillon ;
- des moyens pour commander et permettre une première rotation du bras oscillant (10) autour de l'axe vertical (84), d'une position intiale du godet (4) écartée du filet de matériau liquide (12) vers une position de prélèvement sous le filet de matériau liquide (12) ; et
- une deuxième rotation du bras oscillant (10) autour de l'axe vertical (84) de la position de prélèvement vers une position de repli écartée du filet de matériau liquide (12).

4. Dispositif selon la revendication 3, caractérisé en ce qu'il comprend une pastille fusible (7) bloquant en position initiale le bras (10) et le libérant en rotation par la chute d'une tige lestée (8), lors de la fusion de la pastille (7).

5. Dispositif selon la revendication 4, caractérisé en ce que les moyens de mise en rotation horizontale comprennent un ressort (22) précontraint et monté autour de la tige support verticale (5), une première extrémité supérieure (23) étant fixée à la tige support verticale (5) et une deuxième extrémité inférieure (24) étant fixée au bras oscillant (10), pour permettre la rotation de celui-ci autour de l'axe vertical (84).

6. Dispositif selon la revendication 4, caractérisé en ce que les moyens de mise en rotation comprennent un contrepoids de rotation (21) suspendu et relié horizontalement par l'intermédiaire de poulies (23) par un câble (85) au bras oscillant (10).

7. Dispositif selon la revendication 3, caractérisé en ce que des moyens de commande de la rotation autour de l'axe horizontal (15) comprennent un contrepoids (11) placé à la deuxième extrémité du bras oscillant (10) pour maintenir dans une position haute de remplissage le godet (4) et pour faire osciller le bras oscillant (10) lorsque le godet (4) est rempli du matériau liquide et dégager le bras oscillant (10) d'une pièce fixe (19), libérant ainsi en rotation le bras oscillant (10) jusqu'à la position de repli.

8. Dispositif selon l'une quelconque des revendications 3 à 7, caractérisé en ce que la forme du godet (4) est telle que celui-ci présente deux compartiments.

9. Dispositif pour récupérer le godet (4) de matériau recueilli et solidifié après refroidissement du dispositif selon l'une quelconque des revendications 3 à 8, caractérisé en ce qu'il comprend :
- des moyens de fixation sur le rebord supérieur (3) d'un conteneur (2) et sur lesquels est montée :
- une pince (62) comportant plusieurs griffes (63) maintenues en position de fermeture par des ressorts de rappel et pouvant prendre deux positions verticales différentes qui sont :
. une position haute de repos, les grilles (63) étant fermées ; et
. une position basse de préhension, les grilles (63) étant également fermées, mais ayant été ouvertes lors de la descente de la pince (62) pour saisir le godet (4) par un rebord supérieur (34) du godet (4).

10. Dispositif pour ramener le bras oscillant (10) du dispositif selon l'une quelconque des revendications 4 à 8, dans sa position de prélèvement avant la récupération du godet, et comportant :
- des moyens de fixation sur le rebord supérieur (3) du conteneur (2) avec un indexage angulaire ; et
- une première tige verticale d'entraînement (41) montée pivotante autour d'un axe vertical (44) pour que le bras oscillant (10) effectue une rotation de la position de repli vers la position de prélèvement par entraînement de l'extrémité inférieure (41) de la première tige verticale d'entraînement (41) sur une deuxième tige verticale d'entraînement (43) solidaire du bras oscillant (10).

11. Dispositif selon l'une quelconque des revendications 3 à 10, appliqué au prélèvement d'un échantillon de verre radioactif lors de sa coulée dans un conteneur de stockage définitif (2).

## Patentansprüche

1. Verfahren zur automatischen Probennahme eines Materials, das sich wenigstens vorübergehend in der flüssigen Phase befindet, um in einen Behälter (2) gegossen zu werden und in diesem Behälter (2) eingeschlossen zu werden,
**dadurch gekennzeichnet**,
daß es darin besteht, automatisch folgende Operationen durchzuführen:
- Auffangen der Probe während des Gießens des Materials in den Behälter (2) mit Hilfe einer Vorrichtung nach einem der Ansprüche 3 bis 11, die an dem umgebogenen oberen Rand (3) des Behälters (2) befestigt ist und einen Napf (4) in den dünnen Strahl (12) aus flüssigem Material hält; und
- Bergen der in dem Napf (4) aufgefangenen Probe.

2. Verfahren nach Anspruch 1, bestimmt zur Entnahme einer Probe eines radioaktiven Glases vor seiner definitiven Einschliessung in den Behälter (2), wobei die Vorrichtung zur Entnahme mittels eines Napfes (4) definitiv in dem Behälter (2) zurückgelassen wird, um mit dem vitrifizierten bzw. glasartig gewordenen Produkt eingeschlossen zu werden.

3. Vorrichtung zum Auffangen eines Materials, das sich wenigstens vorübergehend in der flüssigen Phase befindet und in einen Behälter (2) gegossen wird, einen Napf (4) zum Auffangen des Materials umfassend
**dadurch gekennzeichnet**,
daß es zum automatischen Funktionieren umfaßt:
- eine Haltestange (5) ;
- einen Schwenkarm (10), an dessen erstem Ende der Napf (4) befestigt ist, wobei dieser Arm schwenkbar auf der Haltestange (5) angebracht ist, um eine horizontale Achse (15) und eine vertikale Achse der vertikalen Stange (5) herum;
- Befestigungseinrichtungen der Haltestange (5) an dem umgebogenen oberen Rand (3) des Behälters (2), derart, daß sich der Napf (4) unter dem dünnen Gießstrahl (12) befinden und die Probe auffangen kann;
- Einrichtungen, um eine erste Drehungbewegung des Schwenkarms (10) um die vertikale Achse (84) herum zu steuern und zu ermöglichen, aus einer Anfangsstellung des Napfes (4), beabstandet vom dünnen Flüssigmaterialstrahl (12), in eine Entnahmestellung unter dem dünnen Flüssigmaterialstrahl (12); und
- eine zweite Drehbewegung des Schwenkarms (10) um die vertikale Achse (84) herum, aus der Entnahmestellung in eine vom dünnen Flüssigmaterialstrahl (12) beabstandete Rückzugstellung.

4. Vorrichtung nach Anspruch 3, dadurch gekennzeichnet, daß sie eine schmelzbare Tablette (7) umfaßt, die den Arm (10) in der Anfangsstellung blockiert und ihn zur Drehung freigibt durch das Abfallen einer Balast-Stange (8) beim Schmelzen der Tablette (7).

5. Vorrichtung nach Anspruch 4, dadurch gekennzeichnet, daß die Einrichtungen zum Ausführen der horizontalen Drehbewegung eine Feder (22) umfassen, vorgespannt und um die vertikale Haltestange (5) herum angebracht, wobei ein erstes, oberes Ende (23) an der vertikalen Haltestange (5) befestigt ist und ein zweites, unteres Ende (24) an dem Schwenkarm (10) befestigt ist, um dessen Drehung um die vertikale Achse (84) herum zu ermöglichen.

6. Vorrichtung nach Anspruch 4, dadurch gekennzeichnet, daß die Einrichtungen zum Ausführen der horizontalen Drehbewegung ein Rotationsgegengewicht (21) umfassen, aufgehängt und horizontal über Rollen (23) durch ein Kabel (85) mit dem Schwenkarm (10) verbunden.

7. Vorrichtung nach Anspruch 3, dadurch gekennzeichnet, daß die Einrichtungen zum Steuern der Drehung um die horizontale Achse (15) ein Gegengewicht (11) umfassen, angebracht am zweiten Ende des Schwenkarms (10), um den Napf (4) in einer oberen Füllstellung zu halten und um den Schwenkarm (10) zu schwenken, wenn der Napf mit flüssigem Material gefüllt ist, und den Schwenkarm (10) von einem festen Teil (19) freizumachen und so den Schwenkarm (10) zur Drehung in die Rückzugstellung freizugeben.

8. Vorrichtung nach einem der Ansprüche 3 bis 7, dadurch gekennzeichnet, daß die Form des Napfes (4) derartig ist, daß dieser zwei Abteile aufweist.

9. Vorrichtung zum Bergen des Napfes (4) mit aufgefangenem und erstarrtem Material nach Abkühlung der Vorrichtung nach einem der Ansprüche 3 bis 8, dadurch gekennzeichnet, daß sie umfaßt:
- Befestigungseinrichtungen an dem umgebogenen oberen Rand eines Behälters (2), an denen angebracht ist:
- eine Zange (62) mit mehreren Greifern (63), durch Rückstellfedern in Schließstellung gehalten, die zwei verschiedene vertikale Stellungen einnehmen kann, nämlich:
. eine obere Ruhestellung, wobei die Greifer (63) geschlossen sind; und
. eine untere Greifstellung, wobei die Greifer (63) ebenfalls geschlossen sind, aber während der Abwärtsbewegung der Zange geöffnet waren, um den Napf (4) an einem oberen, überstehenden Rand (34) des Napfes (4) zu ergreifen.

10. Vorrichtung um den Schwenkarm (10) der Vorrichtung nach einem der Ansprüche 4 bis 8 in seine Entnahmestellung zu bringen, vor dem Bergen des Napfes, umfassend:
- Befestigungseinrichtungen an dem oberen Rand (3) des Behälters (2) mit einer Winkelindexierung; und
- eine erste vertikale Mitnahmestange (41), drehbar um eine vertikale Achse (44) gelagert, damit der Schwenkarm (10) eine Drehung aus der Rückzugstellung in die Entnahmestellung ausführt, indem das untere Ende der ersten vertikalen Mitnahmestange (41) eine zweite vertikale Mitnahmestange (43) mit sich zieht, die verbunden ist mit dem Schwenkarm (10).

11. Vorrichtung nach einem der Ansprüche 3 bis 10, verwendet zur Entnahme einer Probe eines radioaktiven Glases während seines Gießens in einen Endlagerungsbehälter (2).

## Claims

1. Process for the automatic removal of a material sample which is at least instantaneously in the liquid phase for pouring into a container (2) and which is to be enclosed within the said container (2), characterized in that it consists of collecting the sample during the pouring of the material into the container (2) with the aid of an apparatus according to one of the claims 3 to 11 fixed to the upper rim (3) of the container (2) placing a bucket (4) beneath the liquid material stream (12) and recovering the collected sample in the bucket (4).

2. Process according to claim 1 for removing a radioactive glass sample prior to its definitive confinement in the container (2), the apparatus for sampling by the bucket (4) being definitively left in the container (2), so that it is enclosed therein together with the vitrified product.

3. Apparatus for the automatic collecting of a material sample at least instantaneously in the liquid phase and which is being poured into a container (2) having a bucket (4) for collecting the material, characterized in that it comprises for operating correctly a support rod (5), an oscillating arm (10) at a first end of which is fixed the bucket (4), said arm being mounted in oscillating manner on the support rod (5) about a horizontal axis (15) and a vertical axis of the support rod (5) and means for fixing the support rod (5) to the upper rim (3) of the container (2), so that the bucket (4) can be located beneath the pouring stream (12) and can collect the sample, means for controlling and permitting a first rotation of the oscillating arm (10) about the vertical axis (84) from an initial position of the bucket (4) removed from the liquid material stream (12) towards a sample removal position beneath the liquid material stream (12) and a second rotation of the oscillating arm (10) about the vertical axis (84) from the sample removal position to a folded back position removed from the liquid material stream (12).

4. Apparatus according to claim 3, characterized in that it comprises a meltable pellet (7) blocking in the initial position the arm (10) and freeing it in rotation by the dropping of a weighted rod (8) during the melting of the pellet (7).

5. Apparatus according to claim 4, characterized in that the horizontal rotation means comprise a prestressed spring (22) mounted around the vertical support rod (5), a first upper end (23) being fixed to the vertical support rod (5) and a second lower end (24) being fixed to the oscillating arm (10) in order to permit its rotation about the vertical axis (84).

6. Apparatus according to claim 4, characterized in that the rotating means comprise a rotation counterweight (22) in suspended form and horizontally connected by means of pulleys (23) by a cable (85) to the oscillating arm (10).

7. Apparatus according to claim 3, characterized in that the means for the rotation control about the horizontal axis (15) comprise a counterweight (11) placed at the second end of the oscillating arm (10) to maintain the bucket (4) in the upper filling position and for oscillating the oscillating arm (10) when the bucket (4) is filled with liquid material and free the oscillating arm (10) from a fixed part (19), thus allowing the oscillating arm (10) to rotate up to the folded back position.

8. Apparatus according to any one of the claims 3 to 7, characterized in that the shape of the bucket (4) is such that it has two compartments.

9. Apparatus for recovering the bucket (4) with the collected material which has solidified after cooling using an apparatus for automatically collecting a material sample at least instantaneously in the liquid phase and being poured into a container (2), characterized in that it comprises a bucket (4) for collecting the material, an oscillating arm at a first end of which is fixed the bucket (4), a support rod (5) on which the oscillating arm (10) is mounted in oscillating manner and means for fixing the support rod (5) to the upper rim (3) of the container (2), so that the bucket (4) can be beneath the pouring stream (12) and so as to collect the sample, said recovery apparatus being characterized in that it comprises means for fixing to the upper rim (3) of a container (2) and on which is mounted a gripper (62) having several claws (63) maintained in the closed position by return springs and which can assume two different vertical positions, namely an upper rest position with the claws (63) closed and a lower gripping position, with the claws (63) also closed, but having been opened during the descent of the gripper (62) in order to seize the bucket (4) by its upper rim (34).

10. Apparatus for bringing the oscillating arm (10) of the apparatus according to any one of the claims 4 to 8 into its sampling position prior to the recovery of the bucket and comprising means for fixing to the upper rim (3) of the container (2) with angular indexing and a first vertical drive rod (41) mounted in pivoting manner about a vertical axis (44) so that the oscillating arm (10) performs a rotation from the folded back position to the sampling position by driving the lower end (41) of the first vertical drive rod (41) on a second vertical drive rod (43) integral with the oscillating arm (10).

11. Apparatus according to any one of the claims 3 to 10 applied to the sampling of a radioactive glass sample during its pouring into a definitive storage container (2).
